# EUROPÄISCHE PATENTSCHRIFT

(11) **EP 3 574 864 B1**
(45) Veröffentlichungstag und Bekanntmachung des Hinweises auf die Patenterteilung: **29.05.2024**
(21) Anmeldenummer: 19174292.3
(22) Anmeldetag: 14.05.2019
(51) Int. Cl.: A61B 90/57, F16B 21/16, A61B 90/90

(54) **SELBSTSICHERNDE KUPPLUNGSVORRICHTUNG**
SELF-LOCKING COUPLING DEVICE
DISPOSITIF D'ACCOUPLEMENT AUTO-BLOQUANT

(30) Priorität: 28.05.2018 DE 102018112681
(43) Veröffentlichungstag der Anmeldung: 04.12.2019
(73) Patentinhaber: Karl Storz SE & Co. KG, 78532 Tuttlingen (DE)
(72) Erfinder: STEFAN, Jochen, 78532 Tuttlingen (DE); MERZ, Robin, 78532 Tuttlingen (DE); GRÜNER, Sven, 78532 Tuttlingen (DE)

(56) Entgegenhaltungen:
- DE-A1- 3 401 391
- DE-A1- 19 650 447
- DE-A1-102013 002 818

## Beschreibung

### Technologischer Hintergrund

Die Erfindung betrifft eine Kupplungsvorrichtung zur selbstsichernden mechanischen Verbindung zweier Teile eines Haltesystems insbesondere für medizinische Anwendungen. Ferner betrifft die Erfindung das genannte Haltesystem umfassend die genannte Kupplungsvorrichtung, eine Halte- und eine Klemmvorrichtung, die mit der Kupplungsvorrichtung verbindbar sind.

Ein- oder mehrgliedrige Haltesysteme für medizinische Instrumente, deren Teile mit Kupplungsvorrichtungen verbunden werden können, sind in den unterschiedlichsten Ausgestaltungsformen bekannt. Derartige Haltesysteme sind insbesondere in der minimalinvasiven Chirurgie bei der Instrumentenführung von Vorteil, um einen Operateur, Assistenten oder andere Bedienpersonen von ermüdender Haltearbeit zu entlasten. An dem distalen Ende von einer Haltevorrichtung können chirurgische Instrumente oder Endeffektoren wie Manipulatoren, optische Hilfsmittel wie ein Endoskop, eine Klemme oder dergleichen eingesetzt werden. Dabei ist es für medizinische Anwendungen von großer Bedeutung, dass Endeffektoren und zugehörige Haltevorrichtungen bzw. -segmente steril und keimfrei aufbereitet werden können. Damit lediglich Endeffektoren und Teile des Haltesystems autoklaviert werden können, ist eine Trennung der sterilisierbaren von nicht sterilisierbaren Teilen des Haltesystems notwendig. Diese Trennung kann durch eine Kupplungsvorrichtung erfolgen. Die nicht sterilen Teile eines Haltesystems umfassen hierbei beispielweise für den Betrieb eines Endeffektors notwendigen Elemente wie Antriebseinheit, Stromversorgung wie einen Akku und weitere Elektronik und können mit einem sterilen Drape abgedeckt werden.

Um Teile eines Haltesystems schnell und einfach zu verbinden, kommen üblicherweise lösbare Kupplungsvorrichtungen mit einer Kupplungsbuchse und einen Kupplungsbolzen zum Einsatz. Der Kupplungsbolzen, der auch Bolzenelement genannt wird, sollte sich leicht einführen lassen und durch einfaches Drücken in die finale Rast- bzw. Verriegelungsstellung einrasten. Hierbei sollte nicht auf eine bestimmte Drehung beider Kupplungshälften zueinander geachtet werden müssen.

Für eine Kupplungsvorrichtung ist es nicht nur wichtig, dass ein Haltesegment bzw. die zu kuppelnde Teile einfach verbunden und wieder gelöst werden können, sondern dass das gekuppelte Haltesystem auch unter Last einen sicheren störungsfreien Betrieb ermöglicht. Nachteile bei bekannten Sicherheitsmechanismen für Kupplungsvorrichtungen ergeben sich, wenn Kupplungsvorrichtungen durch zusätzliche Teile beispielsweise durch Splinte, Sicherungsdrähte oder ähnliches gesichert werden. Hier muss der Bediener ein zusätzliches Element zum Sichern der Kupplungsverbindung anbringen und zum Lösen wieder entfernen. Meist kann das Lösen bei derartig gesicherten Kupplungsvorrichtungen nur beidhändig oder mit Werkzeug erfolgen. Ferner besteht der Nachteil, dass z.B. bei der Verwendung von Splinten oder Schraubverbindungen keine Drehbarkeit der beiden Kupplungsteile zueinander gegeben ist. Diese vorgenannten Techniken sind somit nachteilhaft, wenn die Verbindung eine einfache Bedienbarkeit oder ein einhändiges bzw. werkzeugfreies Lösen ermöglichen sollen.

Die Offenlegungsschrift DE 196 50 447 A1 offenbart ein System zur Befestigung von statischen Haltesystemen in Gebäuden. Dazu wird ein bolzenförmiges Einsteckelement fest an einer Decke oder Wand angebracht. Das eigentliche Haltesystem wird mittels einer lösbaren Axialsteckkupplung mit dem Einsteckelement verbunden.

DE 34 01 391 A1 lehrt den Aufbau einer einhändig zu bedienenden Strömungsmittelkupplung. Wird der Kupplungszapfen in die Kupplung eingeführt, drückt er eine federbelastete Schiebehülse zurück, bis Sperrkugeln in seine Ringnut eingreifen und die Kupplung verriegelt ist. Zum Lösen dieser Verbindung wird die Schiebehülse gegen die Federkraft zurückgezogen und die Sperrkugeln geben den Kupplungszapfen frei.

Es ist daher eine Aufgabe eine einfach lösbare Kupplungsvorrichtung bereitzustellen, die eine integrierte Sicherung aufweist. Ferner ist es eine Aufgabe der Kupplungsvorrichtung in der Verriegelungsstellung elektrische Signale und/oder Leistung zwischen den Kupplungsteilen übertragen zu können. Weiterhin besteht der Bedarf, dass ein rotativer Freiheitsgrad der gekuppelten Kupplungsvorrichtung bereitgestellt wird, um einen Endeffektor in eine gewünschte Position optimal positionieren zu können. Nach einer Positionierung des Endeffektors kann eine Haltevorrichtung und darin befindliche Gelenke mit einer über die Kupplungsvorrichtung verbundenen Klemmvorrichtung in der gewünschten Position geklemmt bzw. arretiert werden. Hier es wichtig, dass nach Arretierung der Haltevorrichtung eine Drehbarkeit der Kupplungsteile zueinander ausgeschlossen wird und unter Lastbeaufschlagung möglicherweise auftretende Momente abgefangen werden können.

Eine weitere Aufgabe ist es eine selbstsichernde lasthaltende Kupplungsvorrichtung zur Verfügung zu stellen. Insbesondere besteht der Bedarf trotz im Betrieb auf die Kupplungsvorrichtung wirkender Auszugskräfte, die mehrere Kilo-Newton betragen können, dass die Kupplungsvorrichtung selbstsichernd in einer Verriegelungsstellung gehalten wird und nicht unbeabsichtigt gelöst wird. Hierzu sind halb- bzw. unvollständig verriegelte Zustände auszuschließen. Während bei herkömmlichen Kupplungsvorrichtungen die Gefahr besteht, dass sich eine halbgerastete Kupplungsvorrichtung unter Last löst, soll eine mechanische Eigensicherung zur Verfügung gestellt werden, die bei Lastbeaufschlagung zu einer stärkeren Verriegelung führt.

Weiterhin sollte es eine elektronische Kontrollmöglichkeit geben, ob ein Bolzen ausreichend in die Kupplungsbuchse eingeführt wurde, um bei Zugbelastung nicht mehr herausgezogen werden zu können. Insbesondere wenn sich der Bolzen in einer sogenannten kritischen Stellung befindet, in der der Bolzen unter Last entweder in die Freigabestellung oder in eine Verriegelungsstellung positionierbar ist, soll das Risiko minimiert werden, dass der Bolzen unbeabsichtigt freigegeben wird.

Es ist daher eine weitere Aufgabe der vorliegenden Erfindung Endlagen der Verriegelung bzw. Zwischenpositionen elektronisch bestimmen zu können und damit zu prüfen, ob die Kupplungsvorrichtung belastbar ist. Insbesondere auf dem Gebiet der Medizintechnik mit hohen Sicherheitsanforderungen ist es eine wichtige Aufgabe, eine einfache aber zugleich sichere Kraftübertragung zwischen den zu kuppelnden Teilen von Haltesystemen zu gewährleisten.

### Beschreibung der Erfindung

Auf Grundlage der Erfindung sollen die oben genannten Aufgaben besser als in herkömmlichen mechanischen bzw. elektromechanischen Instrumenten gelöst werden.

Diese Aufgaben werden mit einer erfindungsgemäßen Kupplungsvorrichtung, einem Haltesystem umfassend die genannte Kupplungsvorrichtung gemäß den Merkmalen der unabhängigen Ansprüche gelöst. Bevorzugte Ausgestaltungen der Erfindung ergeben sich aus den Unteransprüchen.

Gemäß einem ersten Aspekt der Erfindung wird eine Kupplungsvorrichtung zur selbstsichernden mechanischen Verbindung zweier Teile eines Haltesystems für medizinische Instrumente bereitgestellt, umfassend ein in eine Buchse einführbares Bolzenelement; wobei die Buchse einen Grundkörper und eine damit fest verbundene Konushülse aufweist, die sich an ihrem freien Ende konisch verengt, wobei eine federbeaufschlagte, klemmkörperführende Käfighülse axial bewegbar in die Konushülse eingesetzt ist; wobei
(a) die Klemmkörper in einer ungekuppelten Stellung (a) mittels der auf die Käfighülse wirkenden Federkraft über die Innenumfangskante der Konushülse radial hinausragend anordenbar sind;
(b) die Klemmkörper in einer Freigabestellung (b) durch Verschieben der Käfighülse gegen die Federkraft entlang der Konusinnenfläche radial nach innen bewegbar sind, um den Innenumfang der Buchse für das Bolzenelement freizugeben; und
(c) die Klemmkörper in einer Verriegelungsstellung (c) in eine Kupplungsnut des Bolzenelementes mittels der Federkraft einrastbar sind;
wobei die in Einführungsrichtung vordere geneigte Kupplungsnutflanke des Bolzenelementes mit der Längsachse des Bolzenelementes einen Winkel α einschließt; wobei die Konusinnenfläche der Konushülse mit der zentralen Hülsenachse einen Winkel β einschließt; und wobei zur selbstsichernden Verbindung der Winkel α größer als der Winkel β ausgebildet ist, um teilweise oder vollständig in die Kupplungsnut eingerastete Klemmkörper unter Beaufschlagung des Bolzenelementes mit einer Zugkraft (Fz) entgegen der Einführungsrichtung in die Verriegelungsstellung (c) zu drücken.

Die Konuswinkel α und β der beiden Kupplungsteile d.h. der Kupplungsnutflanke des Bolzenelementes und des Buchsenkonus sind damit so zueinander gewählt, dass an dem Bolzenelement angreifende Auszugskräfte zu einer stärkeren Verriegelung der Kupplungsvorrichtung führen. In anderen Worten wird mit dem Differenzwinkel γ zwischen der verlängerten Kupplungsnutflanke und der geneigten Konusinnenfläche ein Keil gebildet, der sich in Richtung Buchsenöffnung öffnet, wodurch die Klemmkörper stets in Richtung Buchsenöffnung d.h. radial nach innen und gegen die Einführungsrichtung des Bolzenelementes drängen.

Die gewählten Winkel α und β haben die vorteilhafte Wirkung, dass sobald eine Betriebskraft entgegen der Einführungsrichtung wirkt, die Klemmkörper in die Kupplungsnut bzw. in die Verriegelungsstellung (c) gedrückt werden. Auf diese Weise wird verhindert, dass Auszugskräfte ein unbeabsichtigtes Lösen der Kupplungsvorrichtung erzeugen. Zudem bewirken die Winkel α und β und der damit gebildete Keil, dass auch nur teilweise in die Kupplungsnut eingerastete Klemmkörper durch die Betriebskraft vollständig in die Verriegelungsstellung (c) einrasten. Damit wird eine selbstsichernde Kupplungsvorrichtung ohne zusätzliche Sicherungselemente wie Splinte bereitgestellt, die sich trotz hoher Zugkräfte Fz nicht löst.

Gemäß einem weiteren Aspekt der Erfindung beträgt die Differenz (γ) der Winkel zwischen 1° und 15°. Auch wenn eine geringe Differenz von lediglich 1° für den selbstsichernden Mechanismus ausreicht, können vorzugsweise Differenzen von wenigstens 5° gewählt werden, um mögliche Fertigungstoleranzen und Sicherheitszugaben zu berücksichtigen.

Gemäß einem weiteren Aspekt der Erfindung schließt der Winkel α der vorderen geneigten Kupplungsnutflanke des Bolzenelementes einen Winkel zwischen 5° bis 60°, vorzugsweise 25° ein und der Winkel β der Konusinnenfläche schließt einen Winkel zwischen 10° bis 55°, vorzugsweise 20 ° ein. Wie flach bzw. steil die Winkel gewählt werden hängt zudem von Faktoren wie Lösungskraft und Flächenpressung der Klemmkörper ab.

Gemäß einem weiteren Aspekt der Erfindung weist die Kupplungsvorrichtung ferner ein Schaltelement auf, um die Klemmkörperstellung zu bestimmen und eine kritische Stellung (d) der Klemmkörper auszuschließen, bei der nach einer Beaufschlagung des Bolzenelementes mit einer Zugkraft (Fz) die Klemmkörper entweder in die Freigabestellung (b) oder Verriegelungsstellung (c) drückbar sind; wobei sich die kritische Stellung (d) der Klemmkörper im Bereich zwischen der Innenumfangskante der Konushülse und der Außenumfangskante der Kupplungsnut befindet; und wobei mittels des Schaltelementes die Beaufschlagung des Bolzenelementes mit Zugkraft (Fz) erst freigebbar ist, wenn als Klemmkörperstellung eine Stellung jenseits der kritischen Stellung (d) bestimmt wurde, wobei die Klemmkörper auf dem Bolzenelement teilweise oder vollständig eingerastet in der Verriegelungsstellung (c) positioniert sind.

Wird das Bolzenelement nicht vollständig eingeführt, so können die Klemmkörper unvollständig einrasten und in der genannten kritischen Stellung (d) positioniert sein, so dass die Gefahr besteht, dass die Kupplungsvorrichtung keine Zugkräfte übertragen kann und sich unbeabsichtigt und unkontrolliert löst. Da Bedienpersonen die genannte kritische Stellung (d) nicht feststellen können, wird das Schaltelement, das als Mikroschalter oder Taster oder dergleichen ausgebildet sein kann, eingesetzt. So kann das Schaltelement als Überwachungseinheit dienen und vorteilhafterweise eine Beaufschlagung der Kupplungsvorrichtung mit Zugkraft in der kritischen Stellung ausschließen. Damit ist eine kritische Stellung (d) praktisch nicht mehr möglich und es wird erreicht, dass nur stabile lasthaltende Positionen zur Kraftbeaufschlagung zugelassen werden. Genannte stabile Positionen können einerseits die vollständig eingerastete Klemmkörperposition in der Verriegelungsstellung (c) sein oder teilweise eingerastete Positionen jenseits der kritischen Stellung (d), bei denen die Klemmkörper aufgrund des selbstsichernden Mechanismus bei Zugbelastung in die Verriegelungsstellung drängen.

Gemäß einem weiteren Aspekt der Erfindung weist der Grundkörper eine Mehrzahl an Führungsspalten auf, in der jeweils ein als Schaltstift ausgebildetes Schaltelement mittels der Käfighülse axial bewegbar ist, wobei jeder Schaltstift an seinem freien Ende eine Schaltnocke aufweist, um einen Endschalter zur Freigabe einer Zugkraft (Fz) in Abhängigkeit von der Stellung der Klemmkörper zu betätigen.

Die Führung der Schaltelemente bzw. Schaltstifte in Führungsspalten ermöglichen eine kompakte und zugleich sichere Überwachungseinheit. Jeder Endschalter überwacht, ob die Kupplungsvorrichtung mechanisch auch voll verriegelt. Durch die von Schaltelementen kontrollierte Freigabe einer Zugkraft kann eine über die Kupplungsvorrichtung angeschlossene Haltevorrichtung nicht unbeabsichtigt gelöst werden und damit außer Kontrolle geraten.

Um eine Kraftbeaufschlagung bei einer kritischen Stellung der Kupplungsvorrichtung sicher verhindern zu können, erfolgt die Auswertung der Positionen redundant d.h. mit mindestens zwei Schaltelementen bzw. Sensoren, die vorzugsweise in Bezug auf den Umfang der Buchse gleichmäßig verteilt sind. Die redundante Überwachung mittels einer Mehrzahl von Schaltelementen ist insbesondere im Bereich der Medizintechnik von Bedeutung, um sowohl für die Bedienpersonen als auch für die zu behandelnden Patienten mögliche Gefahren durch unbeabsichtigtes Lösen der Kupplungsvorrichtung ausschließen zu können.

Gemäß einem weiteren Aspekt der Erfindung sind die Endschalter von einem durchsichtigen Abdeckelement umgeben, das wenigstens ein Leuchtelement aufweist, um in Abhängigkeit der Betätigung der Endschalter zu leuchten.

Einerseits dient das Abdeckelement als Gehäuse und schützt vorteilhaft die Schaltmechanik und die Kontaktelemente vor Schmutz und Beschädigung. Andererseits dient das wenigstens eine Leuchtelement unter dem Abdeckelement als Signalanzeige, um in Abhängigkeit der Betätigung des Endschalters zu leuchten. Das Leuchtelement kann ein oder mehrere LEDs umfassen, die neben einem zuordenbaren Endschalter angeordnet ist bzw. sind. Dabei wird das Abdeckelement beispielsweise als Ring aus transparentem oder milchigem Kunststoff hergestellt. Auf diese Weise kann einer Bedienperson angezeigt werden, ob stabile Klemmkörperpositionen bzw. Positionen jenseits der kritischen Stellung (d) vorliegen oder nicht.

Gemäß einem weiteren Aspekt der Erfindung weist die Kupplungsvorrichtung ferner ein mit der Käfighülse verbindbares Betätigungselement auf, wobei die klemmkörperführende Käfighülse mittels des Betätigungselementes in die Freigabestellung entgegen einer Federkraft von Federmitteln bewegbar ist, die zwischen der Konushülse und dem Betätigungselement angeordnet sind.

Mit Hilfe des mit der Käfighülse vorzugsweise fest verbundenen Betätigungselementes kann die Kupplungsvorrichtung ohne Werkzeug von der Verriegelungsstellung (c) in die Freigabestellung (b) zur Demontage gebracht werden. Soll eine Haltevorrichtung in Form eines Armes mit einem Gelenk mit der Kupplungsvorrichtung entkuppelt werden, kann mit Hilfe des Betätigungselements die Demontage einhändig erfolgen. Zum kontrollierten Lösen der Kupplungsvorrichtung sollte die Haltevorrichtung nicht arretiert sein, so dass keine Zugkraft auf die Kupplungsvorrichtung wirkt und ein Kraftfluss zwischen beispielsweise einer Klemmvorrichtung und der Haltevorrichtung nicht unterbrochen werden muss. Dies bedeutet dass sowohl das Lösen als auch das Verbinden einer Haltevorrichtung mit der Kupplungsvorrichtung ohne Kraftbeaufschlagung bei nicht arretiertem bzw. gelöstem Haltearm erfolgt. Wenn das Gelenk des Armes gelöst ist, ist es jedoch notwendig, dass sich eine Hand am distalen Handgriff der Haltevorrichtung befindet, um ein unkontrolliertes Schlackern des Haltearmes zu verhindern. Die andere Hand kann dann das Betätigungselement der Kupplungsvorrichtung zum Lösen bedienen.

Gemäß einem weiteren Aspekt der Erfindung sind das Betätigungselement als Betätigungsring und die Federmittel als Wellfederscheibe ausgebildet.

Die Wellfederscheibe ist einfach zu montieren und wirkt zwischen dem Betätigungselement und der Konushülse.

Gemäß einem weiteren Aspekt der Erfindung weist die Kupplungsvorrichtung ferner eine Auswurffeder auf, um das freigegebene Bolzenelement in der Freigabestellung (b) auszuwerfen, dass ein Wiedereinrasten der Klemmkörper in die Kupplungsnut verhindert wird.

In anderen Worten die Auswurffeder drückt gegen die Einführungsrichtung auf den Auswurfbolzen, um auf diese Weise ein unbeabsichtigtes Wiedereinrasten in die Verriegelungsstellung (c) zu verhindern.

Gemäß einem weiteren Aspekt der Erfindung sind das Bolzenelement und die Buchse frei zueinander drehbar.

Auf diese Weise ist eine Drehung der Kupplungsteile im gelösten Zustand der Haltevorrichtung leichtgängig, damit eine Bedienperson die Haltevorrichtung und angeschlossene Endeffektoren oder dergleichen in eine gewünschte Position bringen kann. Eine zu hohe Leichtgängigkeit beim Drehen kann für die Handhabung und genauer Positionierung nachteilig sein, so dass gemäß einer vorteilhaften Ausbildung der Kupplungsvorrichtung eine gewisse Resthemmung vorgesehen wird.

Dagegen wird unter Last bzw. bei der Übertragung von Klemmkraft zwischen den Kupplungsteilen von einem proximalen Schubelement auf ein distales Schubelement die Drehbarkeit der Kupplungsteile relativ zueinander unterbunden und eine drehfeste Verbindung erzeugt, um die gewählte Position einer angeschlossenen Haltevorrichtung zuverlässig beizubehalten. Auf die Kupplungsvorrichtung wirkende Zugkräfte können beispielsweise von einem proximalen und von einer Spindel antreibbaren Schubelement auf ein distales Schubelement, das in dem Kupplungsbolzen axial geführt wird, eingeleitet werden. Dabei hat das in dem Kupplungsbolzen und dem Haltesegment der Haltevorrichtung geführte Schubelement möglichst wenig Spiel bzw. Winkelversatz entlang der Achse, um zur optimalen Kraftübertragung möglichst wenig Weg durch axiales Spiel bzw. Relativbewegungen zu verlieren. Während das innenliegende Schubelement der Haltevorrichtung in distaler Richtung zur Gelenkklemmung bzw. -arretierung drückt, ist die Kupplungsvorrichtung ausgebildet, das Außenrohr des an die Kupplungsvorrichtung angeschlossenen Haltesegmentes mit der gleichen Kraft gegenzuhalten.

Gemäß einem weiteren Aspekt der Erfindung sind die Klemmkörper als Kugeln, Tonnenwälzkörper oder Zylinderrollen ausgebildet.

Um eine gewisse Resthemmung zu erzeugen können anstelle von Kugeln Tonnenwälzkörper oder Zylinderrollen verwendet werden. Die Klemmkörper können auch bevorzugt als Tonnenwälzkörper ausgebildet sein, um eine vollständige drehfeste Fixierung der Kupplungsvorrichtung unter Last zu unterstützen.

Gemäß einem weiteren Aspekt der Erfindung ist am Ende des Bolzenelementes wenigstens eine Kontaktfläche zum elektrischen Verbinden von einer Signalleitung mit wenigstens einem Kontaktelement des Grundkörpers ausgebildet.

Auf diese Weise kann die Kupplungsvorrichtung in der Verriegelungsstellung elektrische Signale und/oder Leistung zwischen den Kupplungsteilen übertragen. Die wenigstens eine Kontaktfläche ist vorzugsweise ringförmig ausgebildet, um in jeder Stellung des ohne Lastbeaufschlagung frei rotierbaren Bolzenelementes elektrische Signale und/oder Leistung übertragen zu können.

Zur Übertragung von elektrischen Signalen wird vorteilhafterweise ein Kabel in den Kupplungsbolzen eingeführt und die wenigstens eine Leitung mit der vorzugsweise als vergoldeter Schleifring ausgebildeten Kontaktfläche verbunden. Auf diese Weise können elektrische Signale bzw. Leistung zwischen den Kupplungsteilen zuverlässig übertragen werden. Beispielsweise kann ein Schaltsignal zur Betätigung einer Klemmvorrichtung weitergeleitet werden. Zudem können die wenigstens eine oder mehrere elektrische Verbindung(en) auch als Überwachungseinheit fungieren. Sobald die elektrische Verbindung zwischen Bolzen und Buchse hergestellt wird, besteht auch eine einwandfreie und sichere Verbindung zwischen den Kupplungsteilen. Daher kann man mit Hilfe der wenigstens einen Kontaktflächen und zuordenbaren Kontaktelement der Kupplung elektrisch überprüfen, ob die Betriebskraft zur Klemmung bzw. Arretierung der angeschlossenen Haltevorrichtung freigegeben werden kann. Diese elektrische Überprüfung kann zusätzlich oder alternativ zu der oben beschriebenen Überwachungseinheit mit Schaltelementen und Endschaltern eingesetzt werden. Auf diese Weise kann eine volle mechanische Verriegelung sichergestellt werden, bevor die Kupplungsvorrichtung belastet wird.

Gemäß einem weiteren Aspekt werden eine Mehrzahl von Kontaktflächen und eine Mehrzahl von über eine Isolierhülse voneinander isolierten Kontaktelementen bereitgestellt, wobei jede Kontaktfläche des Bolzenelementes mit jeweils einem Kontaktelement des Grundkörpers elektrisch verbindbar ist.

Mit Hilfe einer derartigen elektrischen Schnittstelle können eine Mehrzahl von Signalen bzw. Leistung übertragen werden.

Gemäß einem weiteren Aspekt der Erfindung umfasst eine Kupplungsvorrichtung ein Haltesystem, wobei ein erster Teil des Haltesystems eine Klemmvorrichtung und ein zweiter Teil des Haltesystems eine Haltevorrichtung für medizinische Instrumente ist.

Vorteilhafterweise passen Ausführungsformen der Kupplungsvorrichtung mit Teilen oder Haltesegmenten von Haltevorrichtungen bzw. Klemmvorrichtungen zusammen und funktionieren damit. So kann der Grundkörper der Kupplungsvorrichtung beispielsweise mit dem freien Ende einer Klemmvorrichtung verbunden werden, wobei im Inneren des Haltesegments der Klemmvorrichtung ein bewegbares Schubelement mit einem axial bewegbaren Schubelement der gekuppelten Haltevorrichtung zusammenwirken kann. Weiterhin passen die Ausführungsformen der Kupplungsvorrichtung mit bereits vorhandenen Werkzeugen zusammen.

Die Kupplungsvorrichtung ist vorteilhafterweise so ausgebildet, dass sie schnell die Haltevorrichtung mit der Klemmvorrichtung kuppeln bzw. entkuppeln oder lösen kann. Die Kupplungsvorrichtung ist zu Sicherheitszwecken im Betrieb d.h. unter Last nicht lösbar, auch wenn während des Betriebes der Haltevorrichtung große Kräfte wirken. Die maximale Belastung wird erreicht, wenn die Haltevorrichtung, die vorzugsweise als Haltearm mit einem zentralen Gelenk ausgebildet ist, horizontal ausgestreckt ist und damit die größte Reichweite erhält. In dieser Stellung kann das erfindungsgemäße Haltesystem Haltekräfte beispielsweise zwischen wenigstens 3 kg und 5 kg erreichen. Beispielhafte Reichweiten für das gesamte Haltesystem liegen vorzugsweise zwischen 55 cm und 71 cm.

Für die Anwendungen in sterilen Umgebungen ist es notwendig die Haltevorrichtung bzw. den Haltearm von der Kupplungsvorrichtung zu lösen zu sterilisieren. Nicht zur Sterilisation vorgesehene Teile des Haltesystems wie Kupplungsvorrichtung und Klemmvorrichtung können mit einem geeigneten sterilen Überzug bzw. Drape abgedeckt werden.

Gemäß einem weiteren Aspekt der Erfindung ist der Grundkörper der Buchse so ausgebildet, dass das Ende eines axial verschiebbaren Schubelementes der Klemmvorrichtung in den Grundkörper hineinragend anordenbar ist. Weiterhin ist das Bolzenelement mit einem proximalen Haltesegment der Haltevorrichtung so verbindbar ist, dass das proximale Ende eines in dem Haltesegment und in dem Bolzenelement verschiebbaren Schubelementes über die Frontseite des Bolzenelementes hinausragend anordenbar ist. Ferner ist in der Verriegelungsstellung (c) der Kupplungsvorrichtung mittels des über eine Spindel verschiebbaren Schubelementes der Klemmvorrichtung das proximale Ende des Schubelementes der angekuppelten Haltevorrichtung zur reibschlüssigen Arretierung gegen die Einführungsrichtung des Bolzenelementes axial verschiebbar.

Als Antriebseinheit für die Relativverschiebung des proximalen Schubelementes der Klemmvorrichtung ist vorteilhafterweise ein Spindelantrieb oder dergleichen vorgesehen. Diese Antriebe erlauben eine einfache Steuerung der Relativverschiebung, da sie sowohl von einer Hand als auch mittels eines Motors betätigt werden können. Der Motor kann mittels eines distalen Betätigungselements an der Haltevorrichtung über die elektrischen Verbindungen in der Kupplungsvorrichtung betätigt werden. Auf diese Weise kann die Spindel und damit das Schubelement automatisch mit einem einzigen Betätigungssignal bedient werden. Das Schubelement der Klemmvorrichtung kann mit der Spindel fest verbunden oder einstückig ausgebildet sein. Wird ein Elektromotor zum Spindelantrieb verwendet, weist die Klemmvorrichtung die zugehörige Schaltlogik, die Steuereinheit und Stromversorgung (vorzugsweise ein Akku) auf, wobei der Betrieb der Antriebseinheit nur möglich ist wenn eine Überwachungseinheit wie ein Schaltelement eine vollständige Verriegelungsstellung (c) der Kupplungsvorrichtung feststellen.

Mindestens eine der vorangehenden Aspekte und Ausführungsformen stellt eine oder mehrere Lösungen für die Probleme und Nachteile des Stands der Technik bereit. Andere technische Vorteile der vorliegenden Offenbarung werden für den Fachmann aus der nachfolgenden Beschreibung und den Patentansprüchen ersichtlich sein. Jede beanspruchte Ausführungsform kann mit einer beliebigen anderen beanspruchten Ausführungsform oder beliebigen anderen beanspruchten Ausführungsform technisch kombiniert werden.

### Kurze Beschreibung der Figuren

Weitere Vorteile und Merkmale der Erfindung ergeben sich aus der nachfolgenden Beschreibung von Ausführungsbeispielen anhand der Figuren. Es zeigen:
Fig. 1A eine schematische Schnittansicht einer Kupplungsvorrichtung in der ungekuppelten Stellung (a);
Fig. 1B die in der Fig. 1A gezeigte Kupplungsvorrichtung in der Freigabestellung (b);
Fig. 1C die Kupplungsvorrichtung der Fig. 1A bzw. Fig. 1B in einer Verriegelungsstellung (c);
Fig. 2A eine perspektivische Ansicht einer erfindungsgemäßen Kupplungsvorrichtung in der Freigabestellung (b), wobei Teilschnittansichten Elemente der Buchse veranschaulichen;
Fig. 2B die Kupplungsvorrichtung der Fig. 2A in einer Verriegelungsstellung (c) mit Teilschnittansichten;
Fig. 3A eine Schnittansicht einer erfindungsgemäßen Kupplungsvorrichtung in der kritischen Stellung (d);
Fig. 3B eine Detailansicht der Fig. 3A auf Höhe der Kupplungsnut;
Fig. 4 eine perspektivische Explosionsansicht einer weiteren Ausführungsform der Kupplungsvorrichtung;
Fig. 5 eine schematische perspektivische Ansicht eines Haltesystems mit einer weiteren Ausführungsform einer Kupplungsvorrichtung, die eine proximale Klemmvorrichtung mit einer distalen Haltevorrichtung verbindet und Detailansicht einer Spindel der Klemmvorrichtung,

Die Darstellungen sind schematisch und nicht zwingend maßstabsgerecht. Sie zeigen darüber hinaus nicht alle Details, sondern beschränken sich zum Teil auf die Darstellung der erfindungswesentlichen Einzelheiten sowie weiterer Merkmale, die die Erläuterung und Beschreibung der Erfindung erleichtern. Gleiche Elemente in den unterschiedlichen Figuren werden mit gleichen Bezugszeichen bezeichnet.

### Detaillierte Beschreibung der Figuren

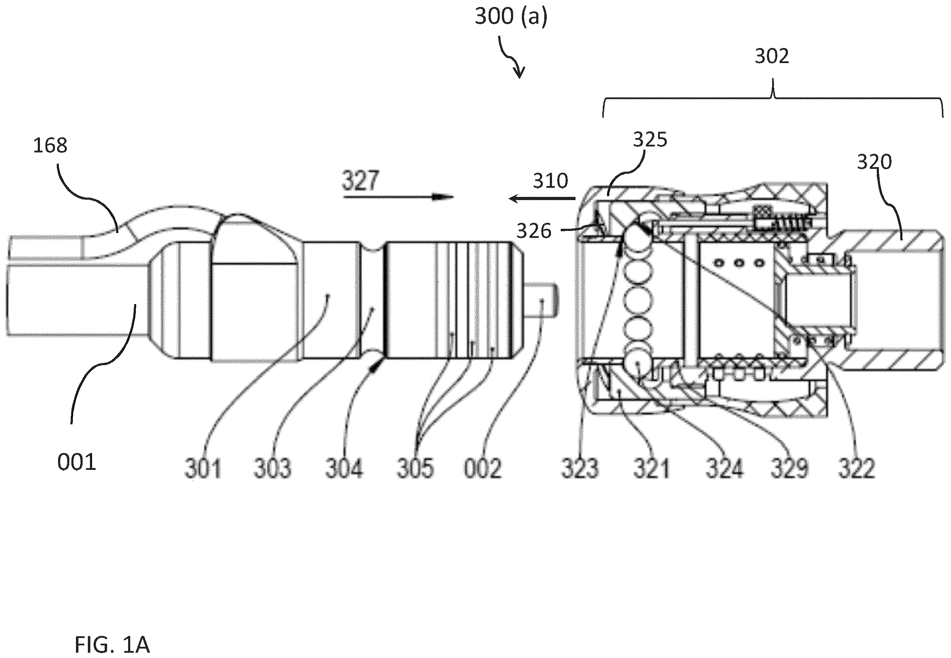

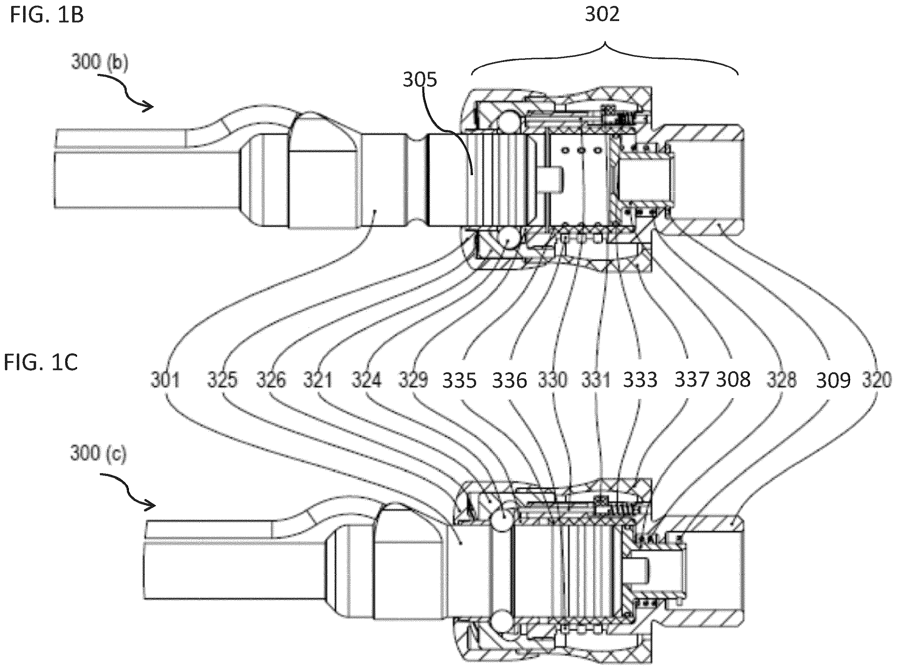

Fig. 1A, Fig. 1B und Fig. 1C zeigen eine Kupplungsvorrichtung 300 gemäß einer Ausführungsform in verschiedenen Stellungen. Fig. 1A zeigt eine ungekuppelte Stellung (a), Fig. 1B eine Freigabestellung (b) und Fig. 1C eine Verriegelungsstellung (c) der Kupplungsvorrichtung 300 (a, b, c). Die Kupplungsvorrichtung 300 besteht im Wesentlichen aus zwei zu verbindenden Teilen, zum einen aus dem Bolzenelement 301 und zum anderen aus der Buchse 302, die ausgelegt ist, das Bolzenelement 301 aufzunehmen. Die wesentlichen Elemente für den selbstsichernden Mechanismus der Kupplungsvorrichtung des Bolzenelementes 301 sind die Kupplungsnut 303 und die an der rechten Nutflanke 304 geneigte Konusfläche des Bolzens 301 und die an den genannten Flächen entlang laufenden Klemmkörper 324.

Das Bolzenelement 301 ist mit einem Haltesegment 001 verbunden, in dem ein axial verschiebbares Schubelement 002 angeordnet ist, das über das Ende des Bolzenelementes 301 hinausragt. Das Bolzenelement 301 kann in Richtung des Pfeiles 327 (Einführungsrichtung) in die Buchse 302 eingeführt bzw. gekuppelt werden und in Auswurfrichtung 310 entkuppelt werden.

Zwischen den Kupplungsteilen sollen nach Kupplung mehrere elektrische Signale und gegebenenfalls Leistung zuverlässig übertragen werden. Hierzu befindet sich am freien Ende des Bolzenelementes 301 eine Mehrzahl von Kontaktflächen 305. Die Kontaktflächen 305 sind ringförmig ausgebildet und bilden eine Schleifringgruppe, um in jeder Stellung des Bolzenelementes elektrische Signale und/oder Leistung übertragen zu können. Ein Kabel 168 wird in den Kupplungsbolzen eingeführt und die jeweiligen Leitungen jeweils mit den einzelnen Kontaktflächen 305 verbunden, um elektrische Signale bzw. Leistung zwischen den Kupplungsteilen übertragen zu können. Beispielsweise kann ein Schaltsignal in der Verriegelungsstellung (c) zur Betätigung einer mit dem Grundkörper 320 verbundenen Klemmvorrichtung 100 weitergeleitet werden, um das axial verschiebbare Schubelement 002 zur Klemmung bzw. Arretierung eines an dem Haltesegment 001 anschließenden Gelenkes zu verschieben.

Die Buchse 302 besteht im Wesentlichen aus einem Grundkörper 320, der mit einer Konushülse 321 verbindbar ist. Der Grundkörper 320 und die dazugehörige Konushülse 321 bilden den unbewegten Teil der Buchse 302. Darin axial beweglich befindet sich die Käfighülse 329, die ihrerseits fest mit einem Betätigungselement 325 in Form eines Betätigungsringes verbunden ist. In der Käfighülse 329 sind kugelförmige Wälzkörper 324 geführt. Sie können auch als Tonnenwälzkörper oder als Zylinderrollen ausgeführt sein.

Die Wälzkörper dienen als Klemmkörper 324. Federmittel 326 in Form einer Wellfederscheibe drücken die Käfighülse 329 zusammen mit dem Betätigungselement 325 ständig gegen die Einführungsrichtung 327 in Richtung des Pfeiles 310 (auf der gezeigten Figur nach links). Durch die federbeaufschlagte Käfighülse 329 werden die Klemmkörper 324 ebenso Richtung Buchsenöffnung (siehe Pfeil 310) gedrückt und laufen an der Konusinnenfläche 322 entlang. Durch die Neigung der Konusinnenfläche 322 werden die Klemmkörper nicht nur nach links (Pfeil 310) sondern auch radial nach innen gedrückt. Mittels der auf die Käfighülse 329 wirkenden Federkraft werden die Klemmkörper 324 damit über die Innenumfangskante 323 der Konushülse 321 radial hinausragend positioniert. In der gezeigten ungekuppelten Stellung (a) ragen die Klemmkörper 324 nicht nur über die Kante 323 der Konushülse 321 hinaus, sondern auch durch die Öffnungen der Käfighülse 329 hindurch.

Wird das freie Bolzenende des Bolzenelementes 301 in die Buchse 302 in die Einführungsrichtung 327 bewegt, befindet sich die Kupplungsvorrichtung 300 zunächst in der in Fig. 1B gezeigten Freigabestellung (b) (Bezugszeichen 300 (b)).

In der ungekuppelten Stellung (a), die in der Fig. 1A gezeigt wird, werden die Klemmkörper 324 soweit es die Käfighülse 329 zulässt, in Richtung des Pfeiles 310 und ins Innere der Buchse gedrückt, so dass die Klemmkörper 324 weit ins Innere der Buchse 302 ragen. Nachdem das Bolzenelement 301 in die Buchse eingeführt wurde, wie in Fig. 1B gezeigt, setzt das Bolzenelement auf die hinausragenden Klemmkörper 324 auf und schiebt diese zusammen mit der Käfighülse 329 in die Einführungsrichtung 327. Dabei können die Klemmkörper 324 entlang der Konusinnenfläche 322 bzw. durch den sich weitenden Konus nach außen laufen, und geben damit den Innendurchmesser der Buchse 302 frei. Auf diese Weise kann das Bolzenelement 301 vollständig in die Buchse 302 eingeschoben werden. Dieser Zustand der vollständigen Verriegelung bzw. der sogenannten Verriegelungsstellung (c) ist in der Fig. 1C gezeigt.

Fig. 1C zeigt die sogenannte Verriegelungsstellung (c), wo das Bolzenelement 301 vollständig in die Buchse 302 eingeführt wurde. In dem dargestellten Zustand der Verriegelungsstellung (c) sind die Klemmkörper 324 weit bzw. maximal in die Kupplungsnut 304 des Bolzenelementes eingerastet. Die Kupplungsnut 303 ist nach dem vollständigen Einschieben des Bolzenelementes 301 so positioniert, dass die Klemmkörper 324 in die Nut gedrückt werden können. Die Federmittel 326 haben dabei die Aufgabe, die Klemmkörper 324 wieder entgegen der Einführungsrichtung in Richtung des Pfeils 310 zu bewegen (hier nach links in Auswurfrichtung). Damit wird die Kupplung verriegelt und die Klemmkörper 324 befinden sich in der Verriegelungsstellung (c).

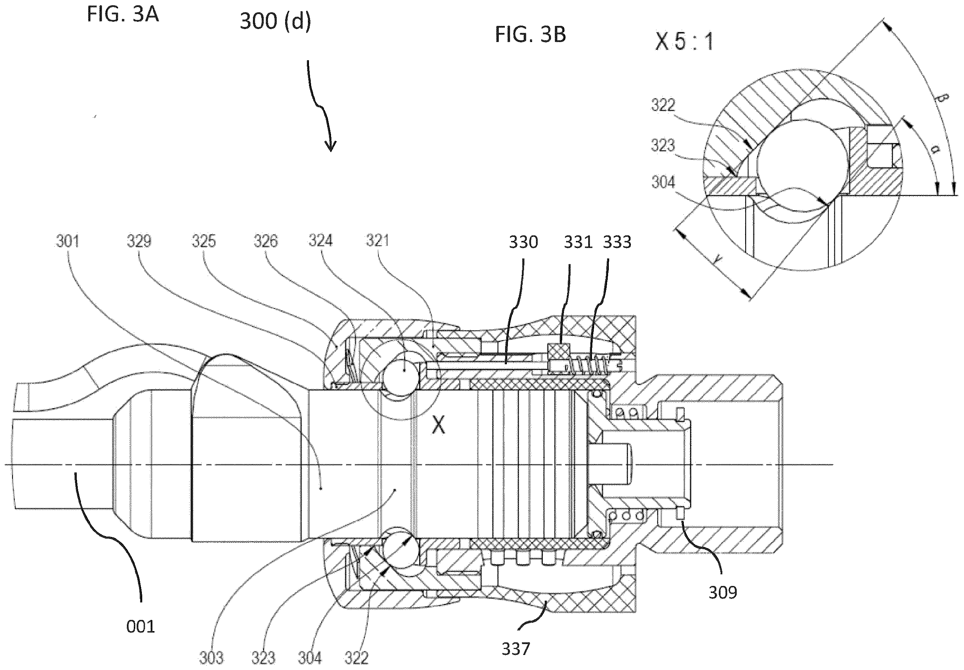

In der gezeigten Verriegelungsstellung (c) können große Zugkräfte Fz auf das Bolzenelement in Richtung 310 bzw. nach links wirken. Damit das Bolzenelement 301 weder unbeabsichtigt freigegeben wird noch die Kupplungsvorrichtung 300 sich unkontrolliert löst, wurden die Winkel α und β an der Kupplungsnutflanke des Bolzenelementes 301 bzw. an der Konusinnenfläche 322 der Buchse 302 so gewählt, dass die in Einführungsrichtung 327 vordere geneigte Kupplungsnutflanke 304 des Bolzenelementes 301 einen größeren Winkel α besitzt als die Neigung β der Konusinnenfläche 322 der Konushülse 321. Dies wird im Detail in Fig. 3B gezeigt.

Ferner zeigt die Fig. 1C elektrische Elemente der Kupplungsvorrichtung 300. In der gezeigten verriegelten Stellung 300 (c) können die Kontaktflächen 305 des Bolzenelementes 301 eine elektrische Verbindung mit entsprechenden Kontaktelementen 336 der Buchse 302 aufnehmen. Die Kontaktelemente 336 sind über eine Isolierhülse 335 voneinander isoliert. Auf diese Weise kann jede Kontaktfläche 305 des Bolzenelementes 301 mit jeweils einem Kontaktelement 336 des Grundkörpers 320 elektrisch verbunden werden. Mit anderen Worten, die Isolierhülse 335 nimmt die Kontaktelemente 336 der Buchse auf und isoliert diese voneinander. Dabei kann jeder geeignete nicht leitende Werkstoff als Isoliermaterial verbaut werden. Auf diese Weise kann nicht nur eine Signalleitung zur Betätigung einer angeschlossenen Klemmvorrichtung 100 sondern auch eine Stromversorgung für distal angeschlossene medizinische Instrumente bereitgestellt werden.

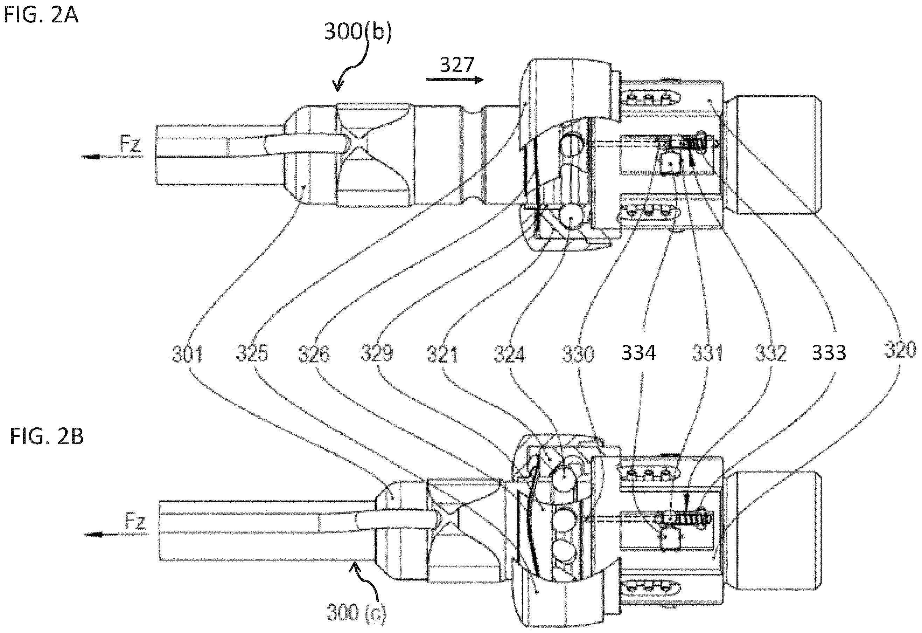

Die Kupplungsvorrichtung 300 weist eine Schaltmechanik mit Schaltelementen 330 auf, um die Klemmkörperstellung zu bestimmen und insbesondere eine kritische Stellung (d) der Klemmkörper 324 auszuschließen, bei der nach einer Beaufschlagung des Bolzenelementes 301 mit einer Zugkraft Fz die Klemmkörper 324 entweder in die Freigabestellung (b) oder Verriegelungsstellung (c) drückbar sind. Figuren 1A-C sowie die perspektivischen Ansichten der Figuren 2A und 2B zeigen eine Schaltmechanik, wobei der Grundkörper 320 eine Mehrzahl von Führungsspalten 332 aufweist, in der jeweils ein als Schaltstift ausgebildetes Schaltelement 330 mittels der Käfighülse 329 in Zusammenwirkung mit einer Schaltfeder 333 axial bewegbar ist (in Figuren 2A und 2B wird mit Bezugszeichen 332 beispielhaft ein Führungsspalt, in dem ein strichliniertes Schaltelement 330 geführt wird, gezeigt). Jeder Schaltstift bzw. Schaltelement 330 weist dabei an seinem freien Ende eine Schaltnocke 331 auf, um einen Endschalter 334 (Bezugszeichen 334 siehe Fig. 2A und 2B) zur Freigabe einer Beaufschlagung mit Zugkraft in Abhängigkeit von der Stellung der Klemmkörper 324 zu betätigen.

Die redundanten Schaltelemente 330 werden mit Hilfe einer Schaltfeder 333 stets gegen die Käfighülse 329 gedrückt, um Fehlbetätigungen des Endschalters 324 in unverriegelten oder unkontrollierbaren teilgerasteten Stellungen auszuschließen. Schnappen die Klemmkörper in der Verrieglungsstellung (c) in die Kupplungsnut 303 vollständig ein, ist die Käfighülse 329 maximal nach links verschoben und die Schaltfeder 333 drückt die jeweiligen Schaltnocken 331 auf die Höhe des Endschalters 334 (siehe Klemmvorrichtung 300(c) in Fig. 2B). Die Schaltmechanik mit ihren Komponenten (vgl. Bezugszeichen 330, 331, 332, 333 und 334) ist in verschiedenen Ansichten bzw. Klemmkörperstellungen in den Figuren 1A, 1B, C, Figuren 2A, 2B sowie Fig. 3A und Fig. 4 veranschaulicht.

Als Gehäuse der Buchse wird ein Abdeckelement bzw. Leuchtring 337 (siehe z.B. Fig. 1B und 1C oder Fig. 3A) bereitgestellt. Dieser Leuchtring 337 hat zum einem die Funktion, die Schaltmechanik zu schützen und zum anderen kann der Leuchtring 337 vorteilhafterweise als Signalanzeige verwendet werden. In diesem Fall wird der Leuchtring 337 aus einem transparenten oder milchigen Kunststoff hergestellt und ein geeignetes Leuchtelement montiert, das den Leuchtring 337 hinterleuchten kann. Dabei kann das Leuchtelement ein oder mehrere LEDs umfassen, die jeweils neben einem zuordenbaren Endschalter 334 angeordnet sind. Auf diese Weise kann einer Bedienperson angezeigt werden, ob stabile Klemmkörperpositionen bzw. Positionen jenseits der kritischen Stellung (d) vorliegen oder nicht.

Nur wenn aufgrund der Klemmkörperposition die Endschalter 334 betätigt werden, ist eine Lastbeaufschlagung mit einer Zugkraft Fz möglich und die relevanten Klemmkörperpositionen können einer Bedienperson vorteilhafterweise durch die genannten Leuchtelemente angezeigt werden. Die Leuchtelemente können hierzu entweder in unterschiedlichen Farben (z.B. grün/rot) oder mit unterschiedlicher Leuchtzeitdauer (z.B. Dauerlicht/Blinklicht) leuchten. Auf diese Weise kann die Schaltmechanik zusammen mit dem Leuchtelement genutzt werden, damit eine Bedienperson eine Kraftbeaufschlagung in Richtung der Zugkraft Fz (siehe Fig. 2A oder 2B) nur aktiviert, wenn sich die Klemmkörper 324 jenseits einer kritischen Stellung bzw. das Bolzenelement 301 fest verriegelt ist.

Zum Lösen der beiden Kupplungsteile des Bolzenelements 301 und der Buchse 302 kann die Verriegelungsstellung (c) über ein Betätigungselement 325 in die Freigabestellung (b) überführt werden. Die Auswurffeder 328 (siehe Fig. 1B und 1C) wird bereitgestellt, um das freigegebene Bolzenelement 301 in der Freigabestellung (b) auszuwerfen, sodass ein Wiedereinrasten der Klemmkörper 324 in die Kupplungsnut 303 verhindert wird. Dabei verhindert der Sicherungsring 309 (siehe Fig. 1B und 1C, Fig. 3B oder Fig. 4), dass der Auswurfsbolzen 308 von der Auswurffeder 328 ganz aus der Kupplungsvorrichtung 300 geschleudert wird.

Fig. 2A und Fig. 2B zeigen den Betätigungsring 325 bzw. das Betätigungselement für die Kupplungsvorrichtung 300 in perspektivischer Teilansicht. Das Betätigungselement 325 kann einfach mit einer Hand in die Einführungsrichtung 327 verschoben werden. In den gezeigten Ansichten wird der Betätigungsring bzw.
-element 325 nach rechts gedrückt, um die Freigabe aus der Verriegelungsstellung (c) zu erzeugen. Von der Käfighülse 329 werden die Klemmkörper 324 dadurch ebenfalls nach rechts geschoben bzw. in Richtung des Pfeiles 327 und können innerhalb des Buchsenkonus entlang der Konusinnenfläche 322 bzw. in der Konushülse 321 radial nach außen bzw. in Richtung des Grundkörpers 320 ausweichen. Dadurch liegen die Klemmkörper 324 nicht mehr in der Kupplungsnut 303 und das Bolzenelement 301 kann in dieser Freigabestellung (b) (siehe z.B. Fig. 2B) aus der Buchse 302 entfernt werden.

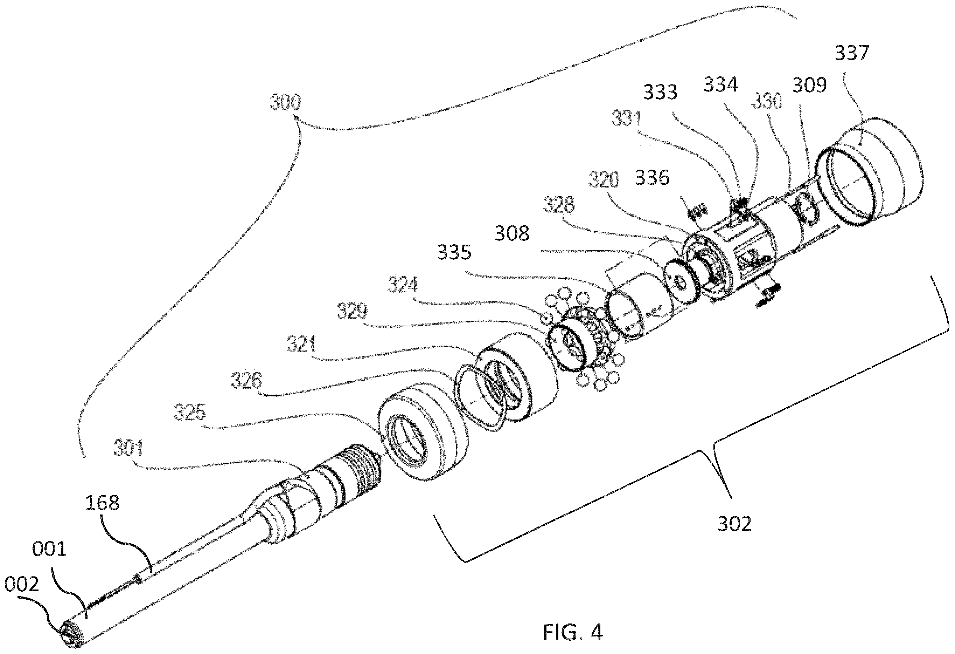

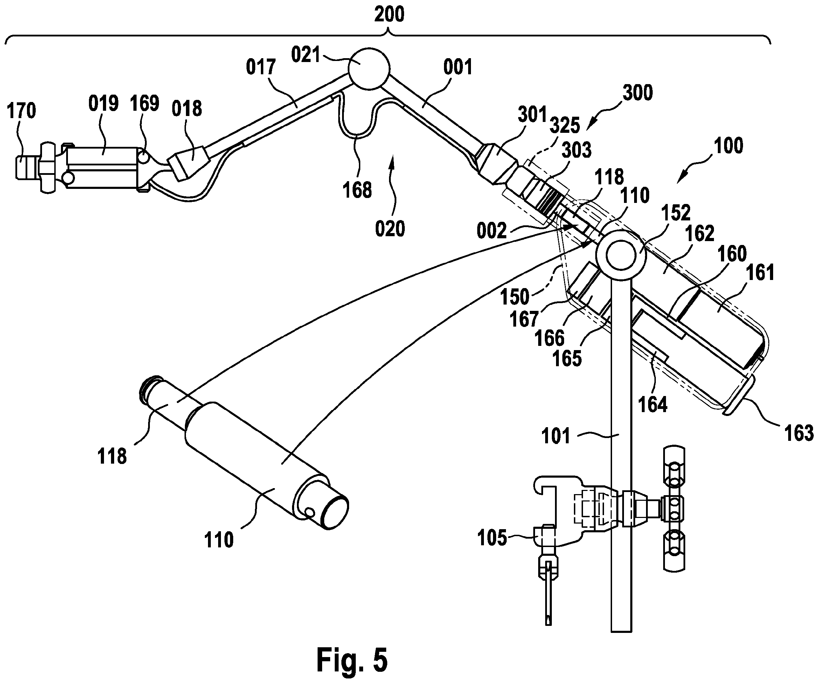

Für das zuverlässige Lösen der Kupplungsvorrichtung 300 drückt eine Auswurffeder 328 (z.B. in Fig. 1 oder Fig. 4 gezeigt) das freigegebene Bolzenelement 301 automatisch ein kleines Stück aus der Kupplungsvorrichtung 300, so dass die Klemmkörper 324 nicht erneut einrasten, wenn der Anwender den Betätigungsring bzw. das Betätigungselement 325 loslässt. Ein Sicherungsring 308 verhindert, dass der Auswurfbolzen zu weit in Richtung Buchsenöffnung gedrückt wird. Wie in den Schnittansichten der Figuren 1A-C und der Explosionsansicht der Fig. 4 gezeigt wird, ist der Auswurfbolzen 308 für das Bolzenelement 301 hohl ausgeführt, um durch die Öffnung ein Schubelement 118 (z.B. in Fig. 5 gezeigt) einer anschließbaren Klemmvorrichtung 100 durchführen zu können.

Fig. 3A zeigt eine Schnittansicht der Kupplungsvorrichtung 300 in der kritischen Stellung (d). und Fig. 3B zeigt ein Detail der Kupplungsnut 303, um die Position der Klemmkörper 324 in der kritischen Stellung (d) zu veranschaulichen. Dabei befindet sich der gezeigte Klemmkörper 324 im Bereich zwischen der Innenumfangskante 323 der Konushülse 321 und der Außenumfangskante der Kupplungsnut 303. Dies bedeutet, der beispielhaft gezeigte Klemmkörper 324 genau an dem Rand zu der geneigten Kupplungsnutflanke 304 positioniert ist.

Die vergrößerte Darstellung gemäß Fig. 3B veranschaulicht, dass der Winkel α der geneigten Kupplungsflanke 304 so ausgebildet ist, dass er größer als der Winkel β ist, der die Konusinnenfläche 322 mit der zentralen Hülsenachse einschließt. Ferner kennzeichnet der Winkel γ die Differenz zwischen den gezeigten Winkeln α und β. Diese Differenz der Neigung der Kupplungsnutflanke 304 und der Neigung der Konusinnenfläche 322 soll vorteilhafterweise zwischen 1° und 15°, vorzugsweise 5° betragen. Die Winkel α und β, die einen Differenzwinkel γ und damit einen sich zur Buchsenöffnung öffnenden Keil aufspannen, bewirken zusammen mit der Federkraft des Federelementes 326, das die Klemmkörper 324 immer in Richtung der Raststellung bzw. Verriegelungsstellung (c) drängen, wenn auf das Bolzenelement 301 eine Zugkraft Fz gegen die Einführungsrichtung 327 wirkt. Die Konuswinkel α und β der beiden Kupplungsteile (301, 302), insbesondere der Kupplungsnutflanke 304 des Bolzenelementes 301 und der Konusinnenfläche 322 der Buchse sind so zueinander gewählt, dass Auszugskräfte Fz zu einer stärkeren Verriegelung der Kupplungsvorrichtung führen. Auf diese Weise kann ein selbstsicherndes Kupplungsverfahren zur Verfügung gestellt werden.

Wie oben beschrieben, wird der potentiell gefährliche kritische Punkt (Stellung (d)) zwischen Verriegelung und Freigabe durch einen Endschalter 324 erkannt und kann somit eine Kraftbeaufschlagung Fz ausschließen und verhindern, dass eine Freigabe in unbeabsichtigter Weise passiert. Werden an die Kupplungsvorrichtung 300 eine Haltevorrichtung 20 und daran zusätzlich Instrumente angeschlossen, können hohe Auszugskräfte Fz während des Betriebes wirken, die je nach Stellung mehrere kN betragen. Durch die selbstsichernde Kupplungsvorrichtung 300 kann sich die erfindungsgemäße Kupplungsvorrichtung 300 jedoch trotz hoher Zugkraft Fz unter Beaufschlagung nicht lösen. Damit werden die hohen Sicherheitsanforderungen bei medizinischen Anwendungen erfüllt.

Die Explosionsdarstellung von Fig. 4 zeigt alle Elemente der Kupplungsvorrichtung 300 insbesondere des Bolzenelementes 301 und der Buchse 302 in perspektivischer Ansicht. In das Bolzenelement 301 wird ein Kabel 168 mit einer Mehrzahl von elektrischen Leitungen eingeführt. Im Inneren des Bolzenelementes 301 und des Haltesegmentes 001 wird mit möglichst wenig Spiel das axial verschiebbare Schubelement 002 geführt.

Ferner wird bezüglich der Buchse 302 illustriert, dass über den gesamten Umfang der Käfighülse 329 eine Mehrzahl von Öffnungen für die jeweiligen Klemmkörper 324 bereitgestellt wird. Zudem wird veranschaulicht, dass der Auswurfbolzen 308 nicht nur hohl ausgestaltet ist, sondern auch eine Flanke aufweist, um in der Freigabestellung (b) mit Hilfe der Auswurffeder 328 Druck auf das Bolzenelement 301 auszuüben. Der Sicherungsring 309 verhindert dabei, dass der Auswurfbolzen 308 von der Auswurffeder 328 vollständig aus der Buchse 302 geschleudert wird.

Wie bereits erläutert können durch die Kupplungsteile 301 und 302 eine Mehrzahl von elektrischen Signalen zuverlässig übertragen werden. Die Isolierhülse 335 ist ausgelegt die Kontaktelemente 336 des Grundkörpers (in Fig. 5 werden hier beispielhaft drei Öffnungen gezeigt) voneinander zu isolieren.

Ferner wird die Schaltmechanik mit drei gleichmäßig im Bezug auf den Umfang des Grundkörpers verteilten Schaltelementen 330, Schaltnocken 331 und Endschaltern 334 illustriert. Jedes Schaltelement wird mit Hilfe einer Schaltfeder 333 stets gegen die Käfighülse 329 gedrückt (vgl. auch vorangehende Figuren wie z.B. Fig. 3A), damit die als Schaltstifte ausgebildeten Schaltelemente 330 nur bei Positionen jenseits der kritische Stellung (d) die jeweiligen Endschalter 324 betätigen.

Es ist ein bedeutender Sicherheitsvorteil, dass mittels der redundanten Anzahl von Schaltelementen und zuordenbaren Endschaltern der Kupplungszustand d.h., ob die Verriegelungsposition (c) bzw. die kritische Klemmkörperstellung (d) vorliegt, zuverlässig bestimmt werden kann.

In der perspektivischen Ansicht der Fig. 5 wird ein Haltesystem 200 und eine perspektivische Detailansicht einer Spindel 110 einer Klemmvorrichtung 100 gezeigt. Das gezeigte Haltesystem 200 verbindet mittels der Kupplungsvorrichtung 300 proximal eine Klemmvorrichtung 100 und distal eine Haltevorrichtung 20. Der Grundkörper 320 der Kupplungsvorrichtung 300 kann bei der Verbindung mit der Klemmvorrichtung 100 auf seiner proximalen Seite zentral ein Schubelement 118 einer Spindel 110 aufnehmen. Die Spindel 110 ist ausgelegt mit Hilfe des Schubelementes 118 das Schubelement 002 der angeschlossenen Haltevorrichtung 20 axial zu verschieben. Durch diese axiale Verschiebung der Schubelemente 118, 002 in eine distale Richtung können Gelenke des Haltesystems 200 zuverlässig geklemmt bzw. arretiert werden. Die Detailansicht der Spindel 110 zeigt, dass das Schubelement 118 fest mit der Spindel 110 verbunden ist. Die Spindel 110 und das Schubelement 118 können auch einstückig ausgebildet sein und als Druckspindel bezeichnet werden.

Fig. 5 zeigt in Bezug auf die Klemmvorrichtung 100 des Haltesystems 200 eine Basissäule 101. Diese Basissäule bzw. Haltesegment 101 ist mit allen bestehenden und handelsüblichen Klemmeinheiten 105 kompatibel, so dass es für medizinische Anwendungen an einem Operationstisch sicher festgeklemmt werden kann. Beispielsweise kann die Basissäule oder das Haltesegment 101 einen Durchmesser von etwa 16 mm aufweisen. In Abhängigkeit von einer besonders großen Nutzlast können auch größere Durchmesser bis zu maximal 2 cm bereitgestellt werden.

Der Befestigungspunkt der Klemmeinheit 105 für das erste Haltesegment 101 ist im proximalen Bereich der Klemmvorrichtung 100 angeordnet. Der proximale Bereich kann sich nahe des Bodens oder eines Operationstisches befinden. Das Haltesystem 200 kann alternativ deckengestützt im Gegensatz zu einem operationstischgestützten System sein. Der distale Bereich des Haltesystems 200 ist der von dem proximalen Bereich entfernt gelegene Bereich. An der distalen Seite der Klemmvorrichtung 100 ist durch die Kupplungsvorrichtung 300 eine Haltevorrichtung 20 in Form eines Haltearms mit Ober- bzw. Unterarm angeschlossen. Die angeschlossene Haltevorrichtung 20 umfasst zwei Haltesegmente 001 und 017, die mit einem Zentralgelenk 21 verschwenkbar miteinander verbunden sind.

Die Klemmvorrichtung 100 weist ein Gehäuse 150 auf. Zur Verschwenkbarkeit des Haltesegments bzw. der Basissäule 101 befindet sich zwischen dem Gehäuse der Klemmvorrichtung 100 und dem Haltesegment 001 ein (Basis-)Gelenk 152, das aufgrund der distal an die Klemmvorrichtung 100 angeschlossenen armähnlichen Haltevorrichtung 20 auch Schultergelenk genannt werden kann.

An dem distalen Haltesegment 017 ist ein Handgriff 019 angeschlossen. An dem freien Ende des Handgriffes 019 bzw. des Haltesystems 200 kann beispielsweise ein medizinisches Instrument befestigt werden. Hierzu weist das distale Ende des Handgriffes 019 eine Kupplungseinheit 170 auf. Diese Kupplungseinheit 170 kann vorzugsweise als Schnellkupplungseinheit wie die bekannte Schnittstelle KSLOCK ausgebildet sein. An eine derartige autoklavierbare Schnellkupplungseinheit 170 können diverse medizinische Instrumente angeschlossen werden wie Mikroscheren, Zangen, Pinzetten, Stanzen oder dergleichen. Ferner kann auch die über die Kupplungsvorrichtung 300 lösbare Haltevorrichtung 20 sterilisiert werden. Für eine Operation sind die Klemmvorrichtung 100 und andere nicht sterilisierbare Elemente mit einem geeigneten Drape abzudecken. Mit einem transparenten Drape können Leuchtanzeigen wie Freigabe zur Lastbeaufschlagung oder andere wichtige Schaltelemente oder dergleichen noch von den Bedienpersonen gesehen bzw. bedient werden.

Der Handgriff 019 weist neben der Verbindung zum Handgelenk 018, das vorzugsweise als Kugelgelenk ausgebildet ist, und der Schnellkupplungseinheit 170 ein Betätigungselement 169 auf. Durch das distale Betätigungselement 169 kann die Antriebseinheit 160 der Klemmvorrichtung 100 aktiviert werden. Das Kontrollsignal für die Aktivierung bzw. Betätigung der dargestellten Spindel 110 kann über das Kabel 168 zu der Antriebseinheit 160 geleitet werden. Das Kabel 168 wird teilweise entlang den Haltesegmenten 017 und 001 geführt. Zwischen dem proximalen Haltesegment 001 und dem distalen Haltesegment 017 wird das Kabel mit etwas Spiel geführt, so dass sich das Zentralgelenk 21 frei bewegen kann. Vorteilhafterweise wir zum Schutz des Kabels die Verdrehbarkeit des Gelenkes 21 auf 340° begrenzt.

Wenn das Kabel 168 zum proximalen Ende des Haltesegmentes 001 geführt wird, tritt es wie in den vorhergehenden Figuren gezeigt in das Bolzenelement 301 ein. Die Kabeleinführung in das Bolzenelement 301 der Kupplungsvorrichtung ermöglicht eine Durchleitung der Steuersignale über die Kupplungsvorrichtung 300 zur Antriebseinheit 160. Die Kupplungsvorrichtung 300 weist im Inneren geeignete Kontaktelemente auf, um das über das Kabel geleitete Signal an die Antriebssteuereinheit 160 weiterzuleiten. Alternativ zur kabelgeführten Aktivierung ist auch eine funkgesteuerte Ansteuerung der Klemmvorrichtung 100 denkbar.

In dem dargestellten Beispiel umfasst die Antriebseinheit 160 für die Spindel 110 einen Elektromotor 161 mit einem Getriebe 162. Die Stromversorgung der Antriebseinheit 160 erfolgt durch einen Akku 163, deren Ladezustand angezeigt werden kann. Der Akku 163 ist in dem Akkuschacht 164 angeordnet und wird über die Akkusteuereinheit 165 gesteuert. Weiterhin befindet sich in dem Gehäuse eine Antriebssteuereinheit 166 sowie eine Schaltlogik 167. Mittels des Getriebes 162 des Elektromotors 161 kann die Spindel 110 angetrieben werden.

Die Auslegung der Schubelemente 002, 118, Haltesegmente 001, 019, 101 und Gelenke 21, 152 des Haltesystems 200 richten sich nach den durchzuleitenden Kräften sowie nach den anzuschließenden Teilen bzw. Instrumenten oder Endeffektoren. Die maximale Reichweite des Haltesystems 200 und gleichzeitig höchste Belastung wird erreicht, wenn der Arm horizontal ausgestreckt ist. In dieser Stellung sollte das erfindungsgemäße Haltesystem 200 Haltekräfte zwischen wenigstens 3 kg und 5 kg erreichen. Beispielhafte Reichweiten für das gesamte Haltesystem liegen zwischen 55 cm und 71 cm. Für die Anwendungen in sterilen Umgebungen ist es notwendig die Haltevorrichtung 020 und Anschlussteile zu sterilisieren. Nicht zur Sterilisation vorgesehene Teile des Haltesystems 200 wie Kupplungsvorrichtung 300 und Klemmvorrichtung 100 können mit einem sterilen Überzug bzw. Drape abgedeckt werden, der eine geeignete Öffnung zum Anschließen der sterilen Haltevorrichtung 20 an die Kupplungsvorrichtung 300 aufweist.

Weiterhin ist die Kupplungsvorrichtung 300 so ausgelegt, dass, sich das Bolzenelement 301 frei um die Hauptachse bewegen kann, solange keine Klemmkraft bzw. Zugkraft Fz zwischen den Kupplungsteilen 301 und 302 übertragen wird. Dieser Rotationsfreiheitsgrad der Kupplungsvorrichtung 300 bzw. des Haltesystems 200 kann vorteilhafterweise bei medizinischen Anwendungen dem Bediener des distalen Instrumentes einen weiteren Freiheitsgrad zur Verfügung stellen.

Durch das Bereitstellen von mehreren Schaltelementen 300 und zuordenbaren Endschaltern 324 wird eine redundante Kontrollmöglichkeit bzw. Überwachung der Klemmkörperpositionen bereitgestellt. Auf diese Weise kann zuverlässig bestimmt werden, ob ein Bolzenelement 301 ausreichend in die Kupplungsbuchse 302 eingeführt wurde, um bei Zugbelastung nicht mehr herausgezogen werden zu können. Insbesondere wenn sich die Klemmkörper 324 in einer sogenannten kritischen Stellung befinden, in der das Bolzenelement unter Last entweder in die Freigabestellung (b) oder in eine Verriegelungsstellung (c) positionierbar ist, kann mit Hilfe der genauen und redundanten Bestimmung der Klemmkörperposition das Risiko minimiert werden, dass der Bolzen 301 unbeabsichtigt freigegeben wird.

Die Erfindung betrifft eine Kupplungsvorrichtung 300 und -verfahren zur selbstsichernden mechanischen Verbindung zweier Teile eines Haltesystems für medizinische Instrumente, umfassend ein in eine Buchse 302 einführbares Bolzenelement 301; wobei die Buchse 302 einen Grundkörper und eine damit fest verbundene Konushülse aufweist, die sich an ihrem freien Ende konisch verengt, wobei eine federbeaufschlagte, klemmkörperführende Käfighülse axial bewegbar in die Konushülse eingesetzt ist und zwischen einer ungekuppelten Stellung (a), einer Freigabestellung (a) und einer Verriegelungsstellung (c) bewegt werden kann. Dabei sind die Klemmkörper 324 in der Verriegelungsstellung (c) in eine Kupplungsnut 303 des Bolzenelementes 301 mittels der Federkraft einrastbar. Die in Einführungsrichtung vordere geneigte Kupplungsnutflanke 304 des Bolzenelementes 301 schließt mit der Längsachse des Bolzenelementes 301 einen Winkel α ein und die Konusinnenfläche 322 der Konushülse 321 mit der zentralen Hülsenachse einen Winkel β. Die Kupplungsvorrichtung 300 kennzeichnet sich dadurch, dass zur selbstsichernden Verbindung der Winkel α größer als der Winkel β ausgebildet ist, um dadurch teilweise oder vollständig in die Kupplungsnut eingerastete Klemmkörper unter Beaufschlagung des Bolzenelementes mit einer Zugkraft (Fz) entgegen der Einführungsrichtung in die Verriegelungsstellung (c) zu drücken.

### Bezugszeichenliste

- 001: (proximales) Haltesegment einer Haltevorrichtung
- 002: (proximales) Schubelement der Haltevorrichtung
- 017: (distales) Haltesegment der Haltevorrichtung
- 018: Handgelenk
- 019: Handgriff
- 020: Haltevorrichtung
- 021: Gelenk der Haltevorrichtung

- 100: Klemmvorrichtung
- 101: Haltesegment der Klemmvorrichtung/Basissäule
- 110: Spindel
- 118: Schubelement/Druckbolzen
- 150: Gehäuse der Klemmvorrichtung
- 152: Gelenk der Klemmvorrichtung/Basisgelenk
- 160: Antriebseinheit
- 161: Elektromotor
- 162: Getriebe
- 163: Akku
- 164: Akkuschacht
- 165: Akkusteuereinheit
- 166: Antriebssteuereinheit
- 167: Schaltlogik
- 168: Kabel
- 169: Betätigungselement für Klemmvorrichtung
- 170: Kupplungseinheit für distales Anschlussteil und/oder ein Instrument

- 200: Haltesystem
- 300: Kupplungsvorrichtung
- 301: Bolzenelement
- 302: Buchse
- 303: Kupplungsnut
- 304: Kupplungsnutflanke
- 305: Kontaktflächen/Kontaktringe
- 308: Auswurfbolzen für das Bolzenelement 301
- 309: Sicherungsring
- 310: Ausführungsrichtung
- 320: Grundkörper
- 321: Konushülse
- 322: Konusinnenfläche
- 323: Innenumfangskante
- 324: Klemmkörper
- 325: Betätigungselement für Kupplungsvorrichtung
- 326: Federmittel
- 327: Einführungsrichtung
- 328: Auswurffeder
- 329: Käfighülse
- 330: Schaltelement
- 331: Schaltnocken
- 332: Führungsspalten
- 333: Schaltfeder
- 334: Endschalter
- 335: Isolierhülse
- 336: Kontaktelemente des Grundkörpers
- 337: Leuchtring
- α: Winkel zwischen Kupplungsnutflanke 304 und Längsachse des Bolzenelementes 301
- β: Winkel der Konusinnenfläche 322 mit Hülsenachse
- γ: Differenz der Winkel α und β
- a: ungekuppelte Stellung
- b: Freigabestellung
- c: Verriegelungsstellung
- d: kritische Stellung
- Fz: Zugkraft gegen die Einführungsrichtung

## Patentansprüche

1. Haltesystem (200) umfassend eine Kupplungsvorrichtung (300) zur selbstsichernden mechanischen Verbindung zweier Teile (100, 20) eines Haltesystems (200) für medizinische Instrumente, umfassend:
ein in eine Buchse (302) einführbares Bolzenelement (301);
wobei die Buchse (302) einen Grundkörper (320) und eine damit fest verbundene Konushülse (321) aufweist, die sich an ihrem freien Ende konisch verengt,
wobei eine federbeaufschlagte, klemmkörperführende Käfighülse (329) axial bewegbar in die Konushülse (321) eingesetzt ist; wobei
(a) Klemmkörper (324) in einer ungekuppelten Stellung (a) mittels der auf die Käfighülse (329) wirkenden Federkraft über die Innenumfangskante (323) der Konushülse (321) radial hinausragend anordenbar sind;
(b) die Klemmkörper (324) in einer Freigabestellung (b) durch Verschieben der Käfighülse (329) gegen die Federkraft entlang der Konusinnenfläche (322) radial nach innen bewegbar sind, um den Innenumfang der Buchse (302) für das Bolzenelement (301) freizugeben; und
(c) die Klemmkörper in einer Verriegelungsstellung (c) in eine Kupplungsnut des Bolzenelementes (301) mittels der Federkraft einrastbar sind;
wobei die in Einführungsrichtung (327) vordere geneigte Kupplungsnutflanke (304) des Bolzenelementes (301) mit der Längsachse des Bolzenelementes (301) einen Winkel α einschließt;
wobei die Konusinnenfläche (322) der Konushülse (321) mit der zentralen Hülsenachse einen Winkel β einschließt; und
wobei zur selbstsichernden Verbindung der Winkel α größer als der Winkel β ausgebildet ist, um teilweise oder vollständig in die Kupplungsnut (303) eingerastete Klemmkörper unter Beaufschlagung des Bolzenelementes (301) mit einer Zugkraft (Fz) entgegen der Einführungsrichtung (327) in die Verriegelungsstellung (c) zu drücken,
wobei ein erster Teil des Haltesystems (200) eine Klemmvorrichtung (100) ist; und
wobei ein zweiter Teil des Haltesystems (200) eine Haltevorrichtung (20) für medizinische Instrumente ist,
wobei der Grundkörper (320) der Buchse (302) so ausgebildet ist, dass das Ende eines axial verschiebbaren Schubelementes (118) der Klemmvorrichtung (100) in den Grundkörper (320) hineinragend anordenbar ist; und
wobei das Bolzenelement (301) mit einem proximalen Haltesegment (001) der Haltevorrichtung (020) so verbindbar ist, dass das proximale Ende eines in dem Haltesegment (001) und in dem Bolzenelement (301) verschiebbaren Schubelementes (002) über die Frontseite des Bolzenelementes (301) hinausragend anordenbar ist; und
wobei in der Verrieglungsstellung (c) der Kupplungsvorrichtung (300) mittels dem über eine Spindel (110) verschiebbaren Schubelement (118) der Klemmvorrichtung (100) das proximale Ende des Schubelementes (002) der angekuppelten Haltevorrichtung (101) zur reibschlüssigen Arretierung gegen die Einführungsrichtung (327) des Bolzenelementes (301) axial verschiebbar ist.

2. Haltesystem nach Anspruch 1, wobei die Differenz (γ) der Winkel (α, β) zwischen 1° und 15° beträgt.

3. Haltesystem nach einem der vorhergehenden Ansprüche, wobei der Winkel α der vorderen geneigten Kupplungsnutflanke (304) des Bolzenelementes (301) einen Winkel zwischen 5° bis 60° einschließt und der Winkel β der Konusinnenfläche (322) einen Winkel zwischen 10° bis 55° einschließt.

4. Haltesystem nach einem der vorhergehenden Ansprüche,
wobei die Kupplungsvorrichtung (300) ferner wenigstens ein Schaltelement (330) aufweist, um die Klemmkörperstellung zu bestimmen und eine kritische Stellung (d) der Klemmkörper (324) auszuschließen, bei der nach einer Beaufschlagung des Bolzenelementes (301) mit einer Zugkraft (Fz) die Klemmkörper (324) entweder in die Freigabestellung (b) oder Verriegelungsstellung (c) drückbar sind;
wobei sich die kritische Stellung (d) der Klemmkörper (324) im Bereich zwischen der Innenumfangskante (323) der Konushülse und der Außenumfangskante der Kupplungsnut (303) befindet; und
wobei mittels des Schaltelementes (330) die Beaufschlagung des Bolzenelementes (301) mit Zugkraft (Fz) erst freigebbar ist, wenn als Klemmkörperstellung eine Stellung jenseits der kritischen Stellung (d) bestimmt wurde, wobei die Klemmkörper (324) auf dem Bolzenelement (301) teilweise oder vollständig eingerastet in der Verriegelungsstellung (c) positioniert sind.

5. Haltesystem nach Anspruch 4, wobei der Grundkörper (320) eine Mehrzahl an Führungsspalten (332) aufweist, in der jeweils ein als Schaltstift ausgebildetes Schaltelement (330) mittels der Käfighülse (329) axial bewegbar ist, wobei jeder Schaltstift an seinem freien Ende eine Schaltnocke (331) aufweist, um einen Endschalter (334) zur Freigabe einer Zugkraft (Fz) in Abhängigkeit von der Stellung der Klemmkörper (324) zu betätigen.

6. Haltesystem nach Anspruch 5, wobei die Endschalter (334) von einem durchsichtigen Abdeckelement (337) umgeben sind, das wenigstens ein Leuchtelement aufweist, um in Abhängigkeit der Betätigung der Endschalter (334) zu leuchten.

7. Haltesystem nach einem der vorhergehenden Ansprüche, wobei die Kupplungsvorrichtung (300) ferner ein mit der Käfighülse (329) verbundenes Betätigungselement (325) aufweist, wobei die klemmkörperführende Käfighülse (329) mittels des Betätigungselementes (325) in die Freigabestellung entgegen einer Federkraft von Federmitteln (326) bewegbar ist, die zwischen der Konushülse (321) und dem Betätigungselement (325) angeordnet sind.

8. Haltesystem nach Anspruch 7, wobei das Betätigungselement (325) als Betätigungsring und die Federmittel (326) als Wellfederscheibe ausgebildet sind.

9. Haltesystem nach einem der vorhergehenden Ansprüche, wobei die Kupplungsvorrichtung (300) ferner eine Auswurffeder (328) aufweist, um das freigegebene Bolzenelement (301) in der Freigabestellung (b) auszuwerfen, dass ein Wiedereinrasten der Klemmkörper (324) in die Kupplungsnut (303) verhindert wird.

10. Haltesystem nach einem der vorhergehenden Ansprüche, wobei das Bolzenelement (301) und die Buchse (302) frei zueinander drehbar sind.

11. Haltesystem nach einem der vorhergehenden Ansprüche, wobei die Klemmkörper (324) als Kugeln, Tonnen- oder Zylinderrollen ausgebildet sind.

12. Haltesystem nach einem der vorhergehenden Ansprüche, wobei am Ende des Bolzenelementes (301) wenigstens eine Kontaktfläche (336) zum elektrischen Verbinden von einer Signalleitung mit wenigstens einem Kontaktelement (336) des Grundkörpers (320) ausgebildet ist.

13. Haltesystem nach Anspruch 12, wobei eine Mehrzahl von Kontaktflächen (305) und eine Mehrzahl von über eine Isolierhülse (335) voneinander isolierten Kontaktelementen (311) bereitgestellt werden und jede Kontaktfläche (305) des Bolzenelementes (301) mit jeweils einem Kontaktelement (336) des Grundkörpers (320) elektrisch verbindbar ist.

## Claims

1. A holding system (200) comprising a coupling device (300) for the self-securing mechanical connection of two parts (100, 20) of a holding system (200) for medical instruments, comprising:
a bolt element (301) which can be inserted into a bushing (302);
wherein the bushing (302) has a main body (320) and a cone sleeve (321) firmly connected thereto, which tapers conically at its free end,
wherein a spring-loaded, clamping body-guiding cage sleeve (329) is inserted axially movably into the cone sleeve (321); wherein
(a) clamping bodies (324) can be arranged so as to protrude radially beyond the inner circumferential edge (323) of the cone sleeve (321) in an uncoupled position (a) by means of the spring force acting on the cage sleeve (329);
(b) the clamping bodies (324) can be moved radially inwards in a release position (b) by displacing the cage sleeve (329) against the spring force along the cone inner surface (322) in order to release the inner circumference of the bushing (302) for the bolt element (301); and
(c) the clamping bodies can be snapped into a coupling groove of the bolt element (301) in a locking position (c) by means of the spring force;
wherein the inclined coupling groove flank (304) of the bolt element (301), at the front in the insertion direction (327), forms an angle α with the longitudinal axis of the bolt element (301);
wherein the cone inner surface (322) of the cone sleeve (321) forms an angle β with the central sleeve axis; and
wherein, for the self-securing connection, the angle α is designed to be larger than the angle β in order to press clamping bodies, which are partially or completely snapped into the coupling groove (303), into the locking position (c) counter to the insertion direction (327) by application of a tensile force (Fz) to the bolt element (301),
wherein a first part of the holding system (200) is a clamping device (100); and
wherein a second part of the holding system (200) is a holding device (20) for medical instruments,
wherein the main body (320) of the bushing (302) is designed such that the end of an axially displaceable pushing element (118) of the clamping device (100) can be arranged to protrude into the main body (320); and
wherein the bolt element (301) can be connected to a proximal holding segment (001) of the holding device (020) such that the proximal end of a pushing element (002), which can be displaced in the holding segment (001) and in the bolt element (301), can be arranged so as to protrude beyond the front side of the bolt element (301); and
wherein, in the locking position (c) of the coupling device (300), the proximal end of the pushing element (002) of the coupled holding device (101) can be axially displaced for frictional locking counter to the insertion direction (327) of the bolt element (301) by means of the pushing element (118) of the clamping device (100),
which can be displaced via a spindle (110).

2. The holding system according to claim 1, wherein the difference (γ) of the angles (α, β) is between 1° and 15°.

3. The holding system according to one of the preceding claims, wherein the angle α of the front inclined coupling groove flank (304) of the bolt element (301) forms an angle of between 5° to 60° and the angle β of the cone inner surface (322) forms an angle of between 10° to 55°.

4. The holding system according to one of the preceding claims,
wherein the coupling device (300) further has at least one switching element (330) in order to determine the clamping body position and to exclude a critical position (d) of the clamping bodies (324), in which after a tensile force (Fz) is applied to the bolt element (301), the clamping bodies (324) can be pressed either into the release position (b) or locking position (c);
wherein the critical position (d) of the clamping bodies (324) is in the region between the inner circumferential edge (323) of the cone sleeve and the outer circumferential edge of the coupling groove (303); and
wherein the application of tensile force (Fz) to the bolt element (301) can only be released by means of the switching element (330) when a position beyond the critical position (d) has been determined as the clamping body position, wherein the clamping bodies (324) are positioned partially or completely snapped on the bolt element (301) in the locking position (c).

5. The holding system according to claim 4, wherein the main body (320) has a plurality of guide columns (332), in each of which a switching element (330) designed as a switching pin can be axially moved by means of the cage sleeve (329), wherein each switching pin has a switching cam (331) at its free end to actuate a limit switch (334) to release a tensile force (Fz) depending on the position of the clamping bodies (324).

6. The holding system according to claim 5, wherein the limit switches (334) are surrounded by a transparent cover element (337) which has at least one lighting element to illuminate depending on the actuation of the limit switches (334).

7. The holding system according to one of the preceding claims, wherein the coupling device (300) further has an actuating element (325) connected to the cage sleeve (329), wherein the clamping body-guiding cage sleeve (329) can be moved into the release position by means of the actuating element (325) counter to a spring force of spring means (326), which are arranged between the cone sleeve (321) and the actuating element (325).

8. The holding system according to claim 7, wherein the actuating element (325) is designed as an actuating ring and the spring means (326) as a wave spring washer.

9. The holding system according to one of the preceding claims, wherein the coupling device (300) further has an ejector spring (328) in order to eject the released bolt element (301) in the release position (b) such that the clamping bodies (324) are prevented from snapping back into the coupling groove (303).

10. The holding system according to one of the preceding claims, wherein the bolt element (301) and the bushing (302) are freely rotatable relative to one another.

11. The holding system according to one of the preceding claims, wherein the clamping bodies (324) are designed as balls, barrel rollers or cylindrical rollers.

12. The holding system according to one of the preceding claims, wherein at the end of the bolt element (301) at least one contact surface (336) is formed for electrically connecting a signal line to at least one contact element (336) of the main body (320).

13. The holding system according to claim 12,
wherein a plurality of contact surfaces (305) and a plurality of contact elements (311) insulated from one another via an insulating sleeve (335) are provided and each contact surface (305) of the bolt element (301) can be electrically connected to a respective contact element (336) of the main body (320).

## Revendications

1. Système de maintien (200) comprenant un dispositif d'accouplement (300) pour la liaison mécanique autobloquante de deux pièces (100, 20) d'un système de maintien (200) pour instruments médicaux, comprenant :
un élément de boulon (301) qui peut être inséré dans une douille (302) ;
dans lequel la douille (302) présente un corps de base (320) et un manchon conique (321) fermement relié à celui-ci, qui se rétrécit de manière conique au niveau de son extrémité libre,
dans lequel un manchon de cage de guidage de corps de serrage à ressort (329) est inséré de manière mobile axialement dans le manchon conique (321) ; dans lequel
(a) des corps de serrage (324) peuvent être agencés pour faire saillie radialement au-delà du bord périphérique interne (323) du manchon conique (321) dans une position non accouplée (a) au moyen de la force du ressort agissant sur le manchon de cage (329);
(b) les corps de serrage (324) peuvent être déplacés radialement vers l'intérieur dans une position de libération (b) en déplaçant le manchon de cage (329) contre la force du ressort le long de la surface interne du cône (322) autour de la circonférence interne de la douille (302) pour que l'élément de boulon (301) se libère ; et
(c) les corps de serrage peuvent être encliquetés dans une rainure d'accouplement de l'élément de boulon (301) dans une position de verrouillage (c) au moyen de la force du ressort ;
dans lequel le flanc de rainure d'accouplement incliné avant (304) de l'élément de boulon (301) dans la direction d'insertion (327) forme un angle α avec l'axe longitudinal de l'élément de boulon (301) ;
dans lequel la surface interne du cône (322) du manchon conique (321) forme un angle β avec l'axe central du manchon ; et
dans lequel, pour la liaison autobloquante, l'angle α est conçu pour être plus grand que l'angle β afin de pousser les corps de serrage qui sont partiellement ou
complètement encliquetés dans la rainure d'accouplement (303) dans la position de verrouillage (c) en sollicitant l'élément de boulon (301) avec une force de traction (Fz) à l'encontre de la direction d'introduction (327),
dans lequel une première pièce du système de maintien (200) est un dispositif de serrage (100) ; et
dans lequel une deuxième pièce du système de maintien (200) est un dispositif de maintien (20) pour instruments médicaux,
dans lequel le corps de base (320) de la douille (302) est conçu de telle sorte que l'extrémité d'un élément de poussée (118) déplaçable axialement du dispositif de serrage (100) peut être agencée pour faire saillie dans le corps de base (320) ; et
dans lequel l'élément de boulon (301) peut être relié à un segment de maintien proximal (001) du dispositif de maintien (020) de telle sorte que l'extrémité proximale d'un élément de poussée (002) qui peut être déplacé dans le segment de maintien (001) et dans l'élément de boulon (301), peut être agencée pour faire saillie au-delà du côté avant de l'élément de boulon (301) ; et
dans lequel, dans la position de verrouillage (c) du dispositif d'accouplement (300),
l'extrémité proximale de l'élément de poussée (002) du dispositif de maintien (101) accouplé peut être déplacée axialement à l'encontre de la direction d'insertion (327) de l'élément de boulon (301) pour un blocage par friction, au moyen de l'élément de poussée (118) du dispositif de serrage (100) qui peut être déplacé par l'intermédiaire d'une broche (110).

2. Système de maintien selon la revendication 1, dans lequel la différence (γ) des angles (α, β) est comprise entre 1° et 15°.

3. Système de maintien selon l'une des revendications précédentes, dans lequel l'angle
α du flanc de la rainure d'accouplement avant incliné (304) de l'élément de boulon (301) comprend un angle compris entre 5° et 60° et l'angle β de la surface interne du cône (322) comprend un angle compris entre 10° et 55°.

4. Système de maintien selon l'une des revendications précédentes,
dans lequel le dispositif d'accouplement (300) présente en outre au moins un élément de commutation (330) pour déterminer la position de corps de serrage et exclure une position critique (d) des corps de serrage (324), dans laquelle, après la sollicitation de l'élément de boulon (301) par une force de traction (Fz), les corps de serrage (324) peuvent être poussés soit dans la position de libération (b), soit dans la position de verrouillage (c) ;
dans lequel la position critique (d) des corps de serrage (324) se situe dans la zone entre le bord périphérique interne (323) du manchon conique et le bord périphérique externe de la rainure d'accouplement (303) ; et
dans lequel la sollicitation de l'élément de boulon (301) par la force de traction (Fz) ne peut être libérée au moyen de l'élément de commutation (330) que lorsqu'une position au-delà de la position critique (d) a été déterminée comme position de corps de serrage, dans lequel les corps de serrage (324) sont positionnés partiellement ou complètement encliquetés sur l'élément de boulon (301) dans la position de verrouillage (c).

5. Système de maintien selon la revendication 4, dans lequel le corps de base (320) présente une pluralité de colonnes de guidage (332), dans chacune desquelles un élément de commutation (330) conçu comme une broche de commutation est mobile axialement au moyen du manchon de cage (329), dans lequel chaque broche de commutation présente au niveau de son extrémité libre une came de commutation (331) pour actionner un interrupteur de fin de course (334) afin de libérer une force de traction (Fz) en fonction de la position des corps de serrage (324).

6. Système de maintien selon la revendication 5, dans lequel les interrupteurs de fin de course (334) sont entourés d'un élément de couvercle transparent (337) qui présente au moins un élément d'éclairage afin d'éclairer en fonction de l'actionnement des interrupteurs de fin de course (334).

7. Système de maintien selon l'une des revendications précédentes, dans lequel le dispositif d'accouplement (300) présente en outre un élément d'actionnement (325) relié au manchon de cage (329), dans lequel le manchon de cage (329) guidant les corps de serrage est mobile dans la position de libération au moyen de l'élément d'actionnement (325), à l'encontre d'une force du ressort de moyens à ressort (326), qui sont agencés entre le manchon conique (321) et l'élément d'actionnement (325).

8. Système de maintien selon la revendication 7, dans lequel l'élément d'actionnement (325) est réalisé sous la forme d'une bague d'actionnement et les moyens à ressort (326) sont réalisés sous la forme d'une rondelle élastique ondulée.

9. Système de maintien selon l'une des revendications précédentes, dans lequel le dispositif d'accouplement (300) présente en outre un ressort d'éjection (328) pour éjecter l'élément de boulon (301) libéré dans la position de libération (b), de sorte que les corps de serrage (324) ne puissent pas s'enclencher de nouveau dans la rainure d'accouplement (303).

10. Système de maintien selon l'une des revendications précédentes, dans lequel l'élément de boulon (301) et la douille (302) peuvent tourner librement l'un par rapport à l'autre.

11. Système de maintien selon l'une des revendications précédentes, dans lequel les corps de serrage (324) sont réalisés sous forme de billes, de rouleaux en forme de tonneau ou de rouleaux cylindriques.

12. Système de maintien selon l'une des revendications précédentes, dans lequel au moins une surface de contact (336) est formée au niveau de l'extrémité de l'élément de boulon (301) pour connecter électriquement une ligne de signal à au moins un élément de contact (336) du corps de base (320).

13. Système de maintien selon la revendication 12,
dans lequel une pluralité de surfaces de contact (305) et une pluralité d'éléments de contact (311) isolés les uns des autres par l'intermédiaire d'un manchon isolant (335) sont prévus et chaque surface de contact (305) de l'élément de boulon (301) peut être connectée électriquement à un élément de contact (336) respectif du corps de base (320).
